# EUROPÄISCHE PATENTSCHRIFT

(11) **EP 0 680 374 B1**
(45) Veröffentlichungstag und Bekanntmachung des Hinweises auf die Patenterteilung: **09.04.1997**
(21) Anmeldenummer: 94905047.0
(22) Anmeldetag: 13.01.1994
(51) Int. Cl.: B01D 53/56, B01J 21/04

(54) **VERFAHREN ZUR KATALYTISCHEN ZERSETZUNG VON REINEM ODER IN GASGEMISCHEN ENTHALTENEM DISTICKSTOFFMONOXID**
CATALYTIC DECOMPOSITION PROCESS OF NITROUS OXIDE PURE OR CONTAINED IN GASEOUS MIXTURES
PROCEDE DE DECOMPOSITION CATALYTIQUE DE L'OXYDE AZOTE PUR OU CONTENU DANS DES MELANGES GAZEUX

(30) Priorität: 21.01.1993 DE 4301470
(43) Veröffentlichungstag der Anmeldung: 08.11.1995
(73) Patentinhaber: BASF AKTIENGESELLSCHAFT, 67056 Ludwigshafen (DE)
(72) Erfinder: FETZER, Thomas, D-67346 Speyer (DE); BUECHELE, Wolfgang, D-67063 Ludwigshafen (DE); WISTUBA, Hermann, D-68529 Mannheim (DE); OTTO, Bernhard, D-67117 Limburgerhof (DE); BUERGER, Gert, D-68199 Mannheim (DE); PIJL, Paul, D-67227 Frankenthal (DE)
(86) Internationale Anmeldenummer: EP9400081
(87) Internationale Veröffentlichungsnummer: WO9416798

(56) Entgegenhaltungen:
- EP-A- 0 210 681
- WO-A-94/02244
- US-A- 4 261 862
- US-A- 4 274 981

## Beschreibung

Die Erfindung betrifft ein Verfahren zur katalytischen Zersetzung von reinem oder in Gasgemischen vorhandenem Distickstoffmonoxid mit einem Katalysator, hergestellt durch Vereinigung von R-Al₂O₄, wobei R für ein Element der I., VII. und VIII. Nebengruppe des Periodensystems der Elemente steht, mit Zinn, Blei, einem Element der II. Haupt- oder Nebengruppe des Periodensystems der Elemente als Oxid oder Salz oder in elementarer Form und Calcinieren bei Temperaturen von 300 bis 1300°C und Drücken von 0,1 bis 200 bar.

Eine Übersicht über die Aktivierungsenergien der katalytischen Zersetzung von Distickstoffmonoxid (Lachgas) an oxidischen Katalysatoren, insbesondere an Mischoxiden, ist aus Catalysis Today 4, 235 bis 251 (1989) bekannt.

Die dort beschriebenen Katalysatoren lassen in Aktivität bzw. Standzeit zu wünschen übrig bzw. enthalten teure Elemente wie Edelmetalle.

Der vorliegenden Erfindung lag daher die Aufgabe zugrunde, den zuvor genannnten Nachteilen abzuhelfen.

Demgemäß wurde ein neues und verbessertes Verfahren zur katalytischen Zersetzung von reinem oder in Gasgemischen enthaltenem Distickstoffmonoxid bei Temperaturen von 200 bis 900°C gefunden, welches dadurch gekennzeichnet ist, daß man einen edelmetallfreien Katalysator, hergestellt durch Vereinigung von CuAl₂O₄ mit Zinn, Blei, einem Element der II. Haupt- oder Nebengruppe des Periodensystems der Elemente als Oxid oder Salz oder in elementarer Form und Calcinieren bei Temperaturen von 300 bis 1300°C und Drücken von 0,1 bis 200 bar, einsetzt.

Das erfindungsgemäße Verfahren läßt sich wie folgt durchführen:

Das erfindungsgemäße Verfahren kann so durchgeführt werden, daß man reines Distickstoffmonoxid oder Distickstoffmonoxid enthaltende Gasgemische oder Distickstoffmonoxid enthaltendes Abgas in einem Ofen oder Wärmetauscher auf die notwendige Reaktortemperatur von in der Regel 200 bis 900°C, bevorzugt 250 bis 800°C, besonders bevorzugt 350 bis 700°C vorheizt und dann durch ein mit dem beschriebenen Katalysator gefülltes Reaktionsrohr leitet. Das Vorheizen des Reaktionsgases kann auch direkt im Reaktionsrohr an einer sich auf Reaktionstemperatur befindlichen vorgeschalteten Inertmaterialschicht erfolgen. Zum Aufheizen des Katalysators und/oder des Inertmaterials kann neben einer externen Wärmequelle auch die Reaktionswärme, die bei der Zersetzung des Distickstoffmonoxids frei wird, verwendet werden.

Distickstoffmonoxid kann in Reinstform, in Gemischen mit Sauerstoff oder Luft, in Gemischen mit Luft, die größere Mengen an Wasser und/oder größere Mengen anderer Stickoxide wie Stickstoffmonoxid und Stickstoffdioxid enthalten oder höherer Konzentrationen von Stickoxiden und anderer Gase wie NOₓ, N₂, O₂, CO, CO₂, H₂O und Edelgase, insbesondere Abgase aus Adipinsäureanlagen, eingesetzt werden, wobei es selektiv in die Elemente Stickstoff und Sauerstoff zersetzt werden kann, ohne daß andere Stickoxide in nennenswertem Umfang in die Elemente zersetzt werden. Der Gehalt an Stickoxiden NOₓ kann in der Regel 0 bis 50 Vol.-%, bevorzugt 1 bis 40 Vol.-%, besonders bevorzugt 10 bis 30 Vol.-% und der N₂O-Gehalt kann in der Regel 0,01 bis 65 Vol.-%, bevorzugt 1 bis 55 Vol.-%, besonders bevorzugt 5 bis 45 Vol.-% betragen. Es gelingt beispielsweise Distickstoffmonoxid in Gemischen mit z.B. 20 % Wasser und 65 % Stickstoffdioxid (NO₂) selektiv in die Elemente zu zersetzen.

Als Katalysatoren eignen sich solche, die durch Vereinigung von CuAl₂O₄ mit dem Element als solchem, Oxiden oder Salzen von Zinn, Blei, einem Element der II. Haupt- oder Nebengruppe des Periodensystems der Elemente und Calcinieren bei Temperaturen von 300 bis 1300°C und Drücken von 0,1 bis 200 bar hergestellt werden können. Diese Katalysatoren sind edelmetallfrei (Ag, Au, Pd, Pt) und weisen eine BET-Oberfläche von 1 bis 350 m²/g auf.

Man kann von einem oxidischen Festkörper, der teilweise oder vollständig - also zu 1 bis 100 Gew.-%, bevorzugt 10 bis 90 Gew.-%, besonders bevorzugt 20 bis 70 Gew.-% - ein Spinell der Zusammensetzung CuAl₂O₄ in einer Al₂O₃-Matrix ist, ausgehen und diesen mit gleicher oder höherer Konzentration von Zinn, Blei, einem Element der II. Haupt- oder Nebengruppe des Periodensystems der Elemente als Oxid oder Salz oder in elementarer Form vermischen und bei Temperaturen von 300 bis 1300°C, bevorzugt 500 bis 1200°C, besonders bevorzugt 600 bis 1100°C und Drücken von 0,1 bis 200 bar, bevorzugt 0,5 bis 10 bar, besonders bevorzugt bei Normaldruck (Atmosphärendruck) calcinieren.

Das Vermischen kann z.B. durch Versprühen, mechanisches Vermischen, Verrühren, Verkneten, des gemahlenen Festkörpers der Zusammensetzung CuAl₂O₄, bevorzugt in Al₂O₃, besonders bevorzugt in γ-Al₂O₃ oder bevorzugt durch Imprägnieren eines ungemahlenen Festkörpers der Zusammensetzung CuAl₂O₄, bevorzugt in Al₂O₃, besonders bevorzugt in γ-Al₂O₃ mit Zinn, Blei, einem Element der II. Haupt- oder Nebengruppe des Periodensystems der Elemente als Oxid oder Salz (z.B. in Lösung) oder in elementarer Form erfolgen.

Die Freisetzung des Kupfers in elementarer oder oxidischer Form führt dadurch zur hochdispersen Verteilung, daß durch den Calcinierungsschritt die Elemente Zinn, Blei, der II. Haupt- oder Nebengruppe des Periodensystems der Elemente in elementarer, oxidischer oder salzartiger Form das Kupfer im Spinell teilweise (zu >50 Mol-%, bevorzugt >70 Mol-%, besonders bevorzugt >90 Mol-%) oder vollständig (zu 100 Mol-%) substituieren, da der entstehende Spinell thermodynamisch stabiler ist als der Ausgangsspinell CuAl₂O₄. Der Kupfer- bzw. Kupferoxidgehalt beträgt im einsatzfertigen Katalysator 0,1 bis 50 Gew.-%, bevorzugt 1 bis 40 Gew.-%, besonders bevorzugt 5 bis 30 Gew.-%.

Als Elemente der II. Haupt- oder Nebengruppe des Periodensystems der Elemente eignen sich oxidische oder salzartige Verbindungen oder das Element als solches (in metallischer Form). Als salzartige Verbindungen seien beispielsweise Carbonate, Hydroxide, Carboxylate, Halogenide und oxidische Anionen wie Nitrite, Nitrate, Sulfite, Sulfate, Phosphite, Phosphate, Pyrophosphate, Halogenite, Halogenate und basische Carbonate, bevorzugt Carbonate, Hydroxide, Carboxylate, Nitrite, Nitrate, Sulfate, Phosphate und basische Carbonate, besonders bevorzugt Carbonate, Hydroxide, basische Carbonate und Nitrate, bevorzugt in der Oxidationsstufe +2 wie Zn²⁺, Mg²⁺, Ca²⁺, Sr²⁺ und Ba²⁺, insbesondere Zn²⁺ und Mg²⁺ oder deren Gemische genannt.

Die Herstellung des Ausgangsoxides der Zusammensetzung CuAl₂O₄, bevorzugt in Form eines Spinelles ist z.B. aus Z. Phys. Chem., 141 (1984), 101 bis 103 bekannt. Vorteilhaft erweist sich die Tränkung eines Al₂O₃-Trägers mit einer löslichen Verbindung wie einem Salz des Kations R, z.B. einem Nitrit, Nitrat, Sulfit, Sulfat, Carbonat, Hydroxid, Carboxylat, Halogenid, Halogenit und Halogenat und anschließender thermischer Zersetzung des Anions zum Oxid. Eine weitere Möglichkeit ist das Mischen einer Verbindung wie einem Salz des Kations R mit einer sauerstoffhaltigen Aluminiumverbindung, z.B. durch Trocken oder in Suspension, insbesondere durch Sprühtrocknung, Verdichtung des Materials, z.B. durch Verkneten, gegebenenfalls durch Zugabe eines geeigneten Verformungshilfsmittels, Formgebung durch Extrudieren, Trocknung und anschließender Calcinierung zur Bildung des Spinells. Die Calciniertemperatur kann zwischen 300 und 1300°C, bevorzugt zwischen 600 und 1000°C, liegen.

Die Dotierung von hochoberflächigen Aluminiumoxidträgern, d.h. die Bildung von Mischoxiden, führt zur Erhöhung der thermischen Beständigkeit des Trägers (z.B. DE-A-34 03 328, DE-A-25 00 548, Appl. Catal. 7, 211 bis 220 (1983), J. Catal. 127, 595 bis 604 (1991)). Zusätzlich können die Fremdionen zur katalytischen Aktivität des Katalysators beitragen. Zur Dotierung können im allgemeinen folgende Elemente herangezogen werden: Alkalimetalle, Erdalkalimetalle, Metalle der seltenen Erden, Sc, Ti, V, Cr, Mn, Fe, Co, Ni, Zn, Y, Zr, B, Si, Ge, Sn, Pb, P, Bi. Der Substitutionsgrad von Aluminiumoxid kann z.B. bei 0,01 bis 20 Gew.-% liegen.

Die Partikelgröße der Kupferoxidkristallite im ungebrauchten Katalysator liegt zwischen 1 und 100 nm, bevorzugt zwischen 3 und 70 nm, besonders bevorzugt zwischen 5 und 50 nm. Die Bestimmung der Partikelgröße kann z.B. durch XRD (X-ray defraction) oder TEM (Transmissionseleketronenmikroskopie) erfolgen.

Die erfindungsgemäßen Katalysatoren enthalten Mesoporen von 2 bis. 20 nm und Makroporen von mehr als 20 nm und BET-Oberflächen zwischen 1 und 350 m²/g, bevorzugt zwischen 10 und 200 m²/g, besonders bevorzugt zwischen 25 und 150 m²/g und die Porosität zwischen 0,01 und 0,8 ml/g.

Die bevorzugt bei dem erfindungsgemäße Verfahren eingesetzten Katalysatoren enthalten im allgemeinen 0,1 bis 50 Gew.-%, insbesondere 2 bis 30 Gew.-% Kupferoxid, bezogen auf das Gewicht des Aluminiumoxids. Das spinellbildende Metall liegt in gleicher oder höherer Konzentration als das Kupfer vor (mol/mol).

Die GHSV liegt in der Regel zwischen 500 und 50000 Nl Gas/l Kat*h, vorzugsweise zwischen 1500 und 20000 Nl Gas/l Kat*h.

### Beispiele

### Durchführung der Distickstoffmonoxid-Zersetzung

a) Als Versuchsapparatur für die adiabatische Verfahrenweise dient ein 800 mm langes Reaktionsrohr aus Hasteloy C, unterteilt in Aufheiz- und Reaktionszone. Der Innendurchmesser beträgt 18 mm. Um den Temperaturverlauf im Rohr messen zu können, wurde ein Innenrohr mit 3,17 mm Außendurchmesser eingesetzt, in dem ein Thermoelement leicht verschoben werden kann. Zur besseren Wärmeübertragung wurde der Reaktor in der Aufheizzone mit Inertmaterial (Steatit) gefüllt.
b) Alternativ kann die Reaktion aber auch unter quasi-isothermen Bedingungen in einem Salzbadreaktor durchgeführt werden. Als Wärmeträger dient eine Salzschmelze aus 53 Gew.-% KNO₃, 40 Gew.-% NaNO₂ und 7 Gew.-% NaNO₃. Die Zersetzung wird in einem 600 mm langen Reaktionsrohr aus Hasteloy C durchgeführt. Der Innendurchmesser beträgt 14 mm. Das Gas wird über eine längere Vorheizstrecke auf Reaktionstemperatur gebracht. Um den Temperaturverlauf im Rohr messen zu können, wurde auch hier ein Innenrohr mit 3,17 mm Außendurchmesser eingesetzt, in dem ein Thermoelement leicht verschoben werden kann.

Getestet wurden jeweils 40 ml Katalysator (Splitt 1,5 bis 2 mm).

Getestet wurde die N₂O-Zersetzung in einem Gasgemisch, das für das Abgas einer Adipinsäureanlage typisch ist.

### Typische Gaszusammensetzung:

| | |
|---|---|
| N₂O: | 23 Vol.-% |
| NO₂: | 17 Vol.-% |
| N₂: | 47 Vol.-% |
| O₂: | 7,5 Vol.-% |
| H₂O: | 3 Vol.-% |
| CO₂: | 2,5 Vol.-% |
| GHSV: | 4000 Nl Gas/l Kat*h |

### Herstellung der Katalysatoren

### Beispiel 1

Eine Mischung aus 284 g Puralox® SCF (Fa. Condea), 166 g Pural® SB (Fa. Condea) und 100 g CuO (Fa. Merck) wurde mit 20 ml Ameisensäure (gelöst in 140 ml H₂O) 0,75 h verknetet, zu 3 mm Strängen extrudiert, getrocknet und 4 h bei 800°C calciniert.

71,4 g des CuAl₂O₄ enthaltenden Aluminiumoxid-Trägers (Wasseraufnahme: 69,1 %) wurden zweimal mit 49 ml einer salpetersauren (pH 3) wäßrigen Lösung, die 32,6 g Zn(NO₃)₂ enthält, imprägniert, daraufhin eine Stunde bei Raumtemperatur belassen. Der imprägnierte Träger wurde bis zur Gewichtskonstanz bei 120°C getrocknet und abschließend 4 h bei 600°C calciniert.

### Beispiel 2

Eine Mischung aus 346 g Puralox® SCF (Fa. Condea), 180 g Pural® SB (Fa. Condea) und 120 g CuO (Fa. Merck) wurde mit 18 ml Ameisensäure (gelöst in 390 ml H₂O) 1 h verknetet, zu 3 mm Strängen extrudiert, getrocknet und 4 h bei 800°C calciniert.

85,2 g des CuAl₂O₄ enthaltenden Aluminiumoxid-Trägers (Wasseraufnahme: 70 %) wurden dreimal mit 47 ml einer salpetersauren (pH 2,5) wäßrigen Lösung, die 45,2 g Mg(NO₃)₂·6 H₂O enthält, imprägniert, daraufhin eine Stunde bei Raumtemperatur belassen. Der imprägnierte Träger wurde bis zur Gewichtskonstanz bei 120°C getrocknet und abschließend 4 h bei 700°C calciniert.

### Beispiel 3

Eine Mischung aus 288,4 g Puralox® SCF (Fa. Condea), 350 g Pural® SB (Fa. Condea) und 140 g CuO (Fa. Merck) wurde mit 25 ml Ameisensäure (gelöst in 530 ml H₂O) 1 h verknetet, zu 3 mm Strängen extrudiert, getrocknet und 4 h bei 800°C calciniert.

65,9 g des CuAl₂O₄ enthaltenden Aluminiumoxid-Trägers (Wasseraufnahme: 60,3 %) wurden zweimal mit 47 ml einer salpetersauren (pH 3,1) wäßrigen Lösung, die 34,7 g Ca(NO₃)₂ enthält, imprägniert, daraufhin eine Stunde bei Raumtemperatur belassen. Der imprägnierte Träger wurde bis zur Gewichtskonstanz bei 120°C getrocknet und abschließend 4 h bei 700°C calciniert.

### Vergleichsbeispiel 1

Gemäß DE-A-40 29 061 wurde ein Katalysator nachgestellt. 150 g handelsüblicher Aluminiumoxid-Träger (BET-Oberfläche 1,7 m²/g; Wasseraufnahme 29,2 Gew.-%) wurde mit 100 ml wäßriger Lösung, die 41,7 g AgNO₃ enthält, imprägniert, daraufhin eine Stunde bei Raumtemperatur stehengelassen. Der imprägnierte Träger wurde bis zur Gewichtskonstanz bei 120°C getrocknet und abschließend 4 h bei 700°C calciniert. Der so erhaltene Katalysator enthielt 14,6 Gew.-% Silber und hatte eine BET-Oberfläche von 1,12 m²/g.

### Vergleichsbeispiel 2

Der in DE-A-35 43 640 bevorzugte Palladiumkatalysator auf alpha-Aluminiumoxid wurde nachgestellt. 200 g alpha-Aluminiumoxid (BET-Oberfläche 20,2 m²/g) wurden mit NaOH imprägniert und bei 120°C getrocknet. Dieser Träger wurde mit 96 ml einer wäßrigen Natriumtetrachloropalladat-II-Lösung, enthaltend 1,29 g Pd, imprägniert, daraufhin drei Stunden bei Raumtemperatur stehengelassen. Der Pd²⁺-haltige Träger wurde zur Reduktion des Pd²⁺ mit Hydrazin behandelt. Anschließend wurde der Katalysator chlorfrei gewaschen und bei 120°C bis zur Gewichtskonstanz getrocknet. Der so erhaltene Katalysator enthielt 0,64 Gew.-% Palladium.

### Vergleichsbeispiel 3

Gemäß DE-A-41 28 629 wurde ein Katalysator nachgestellt. 225 g Pural® SB wurden mit 25 g La(NO₃)₃ und 12,5 g Ameisensäure 3 h verknetet, verstrangt, getrocknet und calciniert. 64,10 g hiervon (BET-Oberfläche 183 m²/g; Wasseraufnahme 76 Gew.-%) wurden mit 50,9 ml wäßriger Lösung, die 17,8 g AgNO₃ enthält, imprägniert, daraufhin eine Stunde bei Raumtemperatur stehengelassen. Der imprägnierte Träger wurde bis zur Gewichtskonstanz bei 120°C getrocknet und abschließend 4 h bei 700°C calciniert. Der so erhaltene Katalysator enthielt 14,5 Gew.-% Silber und hatte eine BET-Oberfläche von 156 m²/g.

### Versuchsergebnisse

### a) Adiabatisches Verfahren

| Katalysator | Laufzeit (h) | Temperatur (°C) | Umsatz (%) |
|---|---|---|---|
| 1 | 1036 | 480 | > 99,9 |
| 2 | 1025 | 485 | > 99,9 |
| 3 | 1013 | 485 | > 99,9 |
| V1 | 150 | 610 | 97,5 |
| V2 | 112 | 640 | 66,5 |
| V3 | 280 | 530 | > 99,9 |

Die Versuchsergebnisse (Katalysatoren 1 bis 3) veranschaulichen, daß die neu entwickelten silberfreien Katalysatoren in einem adiabatischen Verfahren gegenüber dem Stand der Technik (Katalysatoren V1 bis V3) sowohl aktiver als auch stabiler sind.

### b) Isothermes Verfahren

| Katalysator | Laufzeit (h) | Saldbadtemperatur (°C) | N₂O-Umsatz (%) |
|---|---|---|---|
| 1 | 48 | 540 | 98,0 |
| 2 | 48 | 540 | 97,2 |
| 3 | 48 | 540 | 97,4 |
| V3 | 48 | 540 | 41,0 |

Die Versuchsergebnisse (Katalysatoren 1 bis 3) zeigen, daß in einem isothermen Verfahren Aktivitätsunterschiede viel deutlicher erkennbar sind als in einem adiabatischen Verfahren, wo die durch die N₂O-Zersetzung freiwerdende Energie einen hohen Beitrag zur Zersetzung liefert. Die Überlegenheit der neu entwickelten silberfreien Katalysatoren gegenüber dem Stand der Technik (Katalysatoren V1 bis V3) kann durch eine isotherme Reaktionsführung deutlich belegt werden.

## Patentansprüche

1. Verfahren zur katalytischen Zersetzung von reinem oder in Gasgemischen enthaltenem Distickstoffmonoxid bei Temperaturen von 200 bis 900°C und Drücken von 0,1 und 20 bar, dadurch gekennzeichnet, daß man einen edelmetallfreien Katalysator, hergestellt durch Vereinigung von CuAl₂O₄ mit Zinn, Blei, einem Element der II. Haupt- oder Nebengruppe des Periodensystems der Elemente als Oxid oder Salz oder in elementarer Form und Calcinieren bei Temperaturen von 300 bis 1300°C und Drücken von 0,1 bis 200 bar, einsetzt.

2. Verfahren zur katalytischen Zersetzung von reinem oder in Gasgemischen enthaltenem Distickstoffmonoxid nach Anspruch 1, dadurch gekennzeichnet, daß man zur Umsetzung von CuAl₂O₄, Zink, Magnesium, Calcium, Strontium oder Barium als Oxid oder Salz oder in elementarer Form verwendet.

3. Verfahren zur katalytischen Zersetzung von reinem oder in Gasgemischen enthaltenem Distickstoffmonoxid nach Anspruch 1, dadurch gekennzeichnet daß der Katalysator eine BET-Oberfläche von 1 bis 350 m²/g.

4. Verfahren zur katalytischen Zersetzung von reinem oder in Gasgemischen enthaltenem Distickstoffmonoxid nach Anspruch 1, dadurch gekennzeichnet, daß der Katalysator einen CuO-Gehalt von 0,1 bis 50 Gew.-% hat.

5. Verfahren zur katalytischen Zersetzung von reinem oder in Gasgemischen enthaltenem Distickstoffmonoxid nach Anspruch 1, dadurch gekennzeichnet, daß das spinellbildende Metalloxid in der Oxidationsstufe +2 vorliegt.

6. Verfahren zur katalytischen Zersetzung von reinem oder in Gasgemischen enthaltenem Distickstoffmonoxid nach Anspruch 1, dadurch gekennzeichnet, daß die Porosität des Katalysators zwischen 0,01 und 0,8 ml/g liegt.

7. Verfahren zur katalytischen Zersetzung von reinem oder in Gasgemischen enthaltenem Distickstoffmonoxid nach den Anspruch 1, dadurch gekennzeichnet, daß der Gehalt an Stickoxiden (NOₓ) zwischen 0 und 50 Vol.-% liegt.

8. Verfahren zur katalytischen Zersetzung von reinem oder in Gasgemischen enthaltenem Distickstoffmonoxid nach den AnSpruch 1, dadurch gekennzeichnet, daß der N₂O-Gehalt des Gasgemisches zwischen 0,01 und 65 Vol.-% liegt.

## Claims

1. A process for the catalytic decomposition of dinitrogen monoxide which is pure or present in gas mixtures at 200-900°C under 0.1 to 20 bar, which comprises employing a catalyst, which is free of noble metals, prepared by combining CuAl₂O₄ with tin, lead, an element of group IIa or IIb of the Periodic Table of the Elements as oxide or salt or in elemental form, and calcining at 300-1300°C under 0.1-200 bar.

2. A process for the catalytic decomposition of dinitrogen monoxide which is pure or present in gas mixtures as claimed in claim 1, wherein zinc, magnesium, calcium, strontium or barium as oxide or salt or in elemental form is used for the conversion of CuAl₂O₄.

3. A process for the catalytic decomposition of dinitrogen monoxide which is pure or present in gas mixtures as claimed in claim 1, wherein the catalyst has a BET surface area of 1-350 m²/g.

4. A process for the catalytic decomposition of dinitrogen monoxide which is pure or present in gas mixtures as claimed in claim 1, wherein the catalyst has a CuO content of 0.1-50% by weight.

5. A process for the catalytic decomposition of dinitrogen monoxide which is pure or present in gas mixtures as claimed in claim 1, wherein the spinel-forming metal oxide is present in the +2 oxidation state.

6. A process for the catalytic decomposition of dinitrogen monoxide which is pure or present in gas mixtures as claimed in claim 1, wherein the porosity of the catalyst is 0.01-0.8 ml/g.

7. A process for the catalytic decomposition of dinitrogen monoxide which is pure or present in gas mixtures as claimed in claim 1, wherein the content of nitrogen oxides (NOₓ) is 0-50% by volume.

8. A process for the catalytic decomposition of dinitrogen monoxide which is pure or present in gas mixtures as claimed in claim 1, wherein the N₂O content of the gas mixture is 0.01-65% by volume.

## Revendications

1. Procédé de décomposition catalytique de l'oxyde azoteux ou protoxyde d'azote pur ou contenu dans des mélanges de gaz à des températures de 200 à 900°C et sous des pressions de 0,1 à 20 bars, caractérisé en ce que l'on utilise un catalyseur dépourvu de métal noble, préparé par la réunion de CuAl₂O₄ et de l'étain, du plomb, d'un élément du second groupe principal ou sous-groupe du système périodique des éléments, sous forme d'oxyde ou de sel, ou sous forme élémentaire et la calcination à des températures de 300 à 1300°C et sous des pressions de 0,1 à 200 bars.

2. Procédé de décomposition catalytique de l'oxyde azoteux ou protoxyde d'azote pur ou contenu dans des mélanges de gaz suivant la revendication 1, caractérisé en ce qu'en vue de la réaction du CuAl₂O₄, on utilise le zinc, le magnésium, le calcium, le strontium ou le baryum sous la forme d'oxyde ou de sel ou sous la forme élémentaire.

3. Procédé de décomposition catalytique de l'oxyde azoteux ou protoxyde d'azote pur ou contenu dans des mélanges de gaz suivant la revendication 1, caractérisé en ce que le catalyseur possède une surface BET qui varie de 1 à 350 m²/g.

4. Procédé de décomposition catalytique de l'oxyde azoteux ou protoxyde d'azote pur ou contenu dans des mélanges de gaz suivant la revendication 1, caractérisé en ce que le catalyseur possède une teneur en CuO qui varie de 0,1 à 50% en poids.

5. Procédé de décomposition catalytique de l'oxyde azoteux ou protoxyde d'azote pur ou contenu dans des mélanges de gaz suivant la revendication 1, caractérisé en ce que l'oxyde de métal formateur de spinelle se présente à l'état d'oxydation +2.

6. Procédé de décomposition catalytique de l'oxyde azoteux ou protoxyde d'azote pur ou contenu dans des mélanges de gaz suivant la revendication 1, caractérisé en ce que la porosité du catalyseur varie de 0,01 à 0,8 ml/g.

7. Procédé de décomposition catalytique de l'oxyde azoteux ou protoxyde d'azote pur ou contenu dans des mélanges de gaz suivant la revendication 1, caractérisé en ce que la teneur en oxydes d'azote (NOₓ) varie de 0 à 50% en volume.

8. Procédé de décomposition catalytique de l'oxyde azoteux ou protoxyde d'azote pur ou contenu dans des mélanges de gaz suivant la revendication 1, caractérisé en ce que la teneur en N₂O du mélange de gaz varie de 0,01 à 65% en volume.
